# EUROPEAN PATENT APPLICATION

(11) **EP 1 655 379 A1**
(43) Date of publication of application: **10.05.2006**
(21) Application number: 05004671.3
(22) Date of filing: 03.03.2005
(51) Int. Cl.: C12Q 1/68

(54) **Method, apparatus and kit for analyzing sample nucleic acid**

(30) Priority: 08.11.2004 JP 2004324138
(71) Applicant: Hitachi, Ltd., Tokyo 100-8280 (JP)
(72) Inventor: Nakashima, Yukie, c/o Hitachi, Ltd., Chiyoda-ku, Tokyo 100-8220 (JP); Nagai, Keiichi, c/o Hitachi, Ltd., Chiyoda-ku, Tokyo 100-8220 (JP)
(74) Representative: Strehl Schübel-Hopf & Partner

(57) **Abstract**

The present invention relates to a technique for detecting the presence or absence of any mutation in a sample nucleic acid having plural possible mutation sites, which is necessary for identification of a cancerous cell.

SEQUENCE LISTING

<110> Hitachi LTD.

<120> Method for analyzing sample nucleic acid, Apparatus for
analyzing nucleic acid and Kit for analyzing sample
nucleic acid

<130> 310400779 US01

<150> JP 2004-324138
<151> 2004-11-05

<160> 60

<170> PatentIn Ver. 2.1

<210> 1
<211> 27
<212> DNA
<213> Artificial Sequence

<220>
<223> Inventor: Nakashima, Yukie; Nagai, Keiichi

<220>
<223> Description of Artificial Sequence:Synthetic DNA (primer)

<400> 1
cgtcaaggca ctcttgccta cgccacc 27

<210> 2
<211> 27
<212> DNA
<213> Artificial Sequence

<220>
<223> Description of Artificial Sequence:Synthetic DNA (primer)

<220>
<221> misc_feature
<222> (27)... (27)
<223> n stands for any base

<400> 2
cgtcaacgca ctcttcccta cgccacn 27

<210> 3
<211> 26
<212> DNA
<213> Artificial Sequence

<220>
<223> Description of Artificial Sequence:Synthetic DNA (primer)

<400> 3
cgtcaaggca ctcttgccta cgccac 26

<210> 4
<211> 25
<212> DNA
<213> Artificial Sequence

<220>
<223> Description of Artificial Sequence:Synthetic DNA (primer)

<220>
<221> misc_feature
<222> (26)... (26)
<223> n stands for any base

<400> 4
cgtcaacgca ctcttcccta cgccan 26

<210> 5

<211> 23
<212> DNA
<213> Artificial Sequence

<220>
<223> Description of Artificial Sequence:Synthetic DNA (primer)

<400> 5
cgtcaaggca ctcttgccta cgc                                              23


<210> 6
<211> 23
<212> DNA
<213> Artificial Sequence

<220>
<223> Description of Artificial Sequence:Synthetic DNA (primer)

<220>
<221> misc_feature
<222> (23)... (23)
<223> n stands for any base

<400> 6
cgtcaacgca ctcttcccta cgn                                              23


<210> 7
<211> 31
<212> DNA
<213> Artificial Sequence

<220>
<223> Description of Artificial Sequence:Synthetic DNA (primer)

<400> 7
ctatctgagc agcgctcatg gtgggggcag c                                     31

<210> 8
<211> 31
<212> DNA
<213> Artificial Sequence

<220>
<223> Description of Artificial Sequence:Synthetic DNA (primer)

<220>
<221> misc_feature
<222> (31)... (31)
<223> n stands for any base

<400> 8
ctatctgagc agagcacatg gtgggggcag n                                31


<210> 9
<211> 27
<212> DNA
<213> Artificial Sequence

<220>
<223> Description of Artificial Sequence:Synthetic DNA (primer)

<400> 9
tggtgaggat gggcctccgg ttcatgc                                     27


<210> 10
<211> 28
<212> DNA
<213> Artificial Sequence

<220>
<223> Description of Artificial Sequence:Synthetic DNA (primer)

<220>
<221> misc_feature
<222> (28)... (28)

<223> n stands for any base

<400> 10
tggtgaggat gggtctcagg ttcatgcn                                        28


<210> 11
<211> 27
<212> DNA
<213> Artificial Sequence

<220>
<223> Description of Artificial Sequence:Synthetic DNA (primer)

<400> 11
tggtgaggat gggcctccgg ttcatgc                                         27


<210> 12
<211> 27
<212> DNA
<213> Artificial Sequence

<220>
<223> Description of Artificial Sequence:Synthetic DNA (primer)

<220>
<221> misc_feature
<222> (27)... (27)
<223> n stands for any base

<400> 12
tggtgaggat gggtctcagg ttcatgn                                         27


<210> 13
<211> 28
<212> DNA
<213> Artificial Sequence

<220>
<223> Description of Artificial Sequence:Synthetic DNA (primer)

<400> 13
gtgtgatgat ggtgaggatg ggcctccg                                        28


<210> 14
<211> 28
<212> DNA
<213> Artificial Sequence

<220>
<223> Description of Artificial Sequence:Synthetic DNA (primer)

<220>
<221> misc_feature
<222> (28)...(28)
<223> n stands for any base

<400> 14
gtgtgatgat ggtgaggatg ggtctccn                                        28


<210> 15
<211> 27
<212> DNA
<213> Artificial Sequence

<220>
<223> Description of Artificial Sequence:Synthetic DNA (primer)

<400> 15
gtgtgatgat ggtgaggatg ggcctcc                                         27


<210> 16
<211> 27
<212> DNA
<213> Artificial Sequence

<220>
<223> Description of Artificial Sequence:Synthetic DNA (primer)

<220>
<221> misc_feature
<222> (27)... (27)
<223> n stands for any base

<400> 16
gtgtgatgat ggtgaggatg ggtctcn 27

<210> 17
<211> 27
<212> DNA
<213> Artificial Sequence

<220>
<223> Description of Artificial Sequence:Synthetic DNA (primer)

<400> 17
gtctctccca ggacaggcac aaacacg 27

<210> 18
<211> 27
<212> DNA
<213> Artificial Sequence

<220>
<223> Description of Artificial Sequence:Synthetic DNA (primer)

<220>
<221> misc_feature
<222> (27)... (27)
<223> n stands for any base

<400> 18
gtctctctca ggacaggcac aaacacn 27

<210> 19
<211> 26
<212> DNA
<213> Artificial Sequence

<220>
<223> Description of Artificial Sequence:Synthetic DNA (primer)

<400> 19
gtctctccca ggacaggcac aaacac                                          26


<210> 20
<211> 26
<212> DNA
<213> Artificial Sequence

<220>
<223> Description of Artificial Sequence:Synthetic DNA (primer)

<220>
<221> misc_feature
<222> (26)... (26)
<223> n stands for any base

<400> 20
gtctctctca ggacaggcac aaacan                                          26


<210> 21
<211> 27
<212> DNA
<213> Artificial Sequence

<220>
<223> Description of Artificial Sequence:Synthetic DNA (primer)

<400> 21

gcggagattc tcttcctctg tgcgccg                                          27

<210> 22
<211> 27
<212> DNA
<213> Artificial Sequence

<220>
<223> Description of Artificial Sequence:Synthetic DNA (primer)

<220>
<221> misc_feature
<222> (27)...(27)
<223> n stands for any base

<400> 22
gcggagattc tcttcgtctg tgtgccn                                          27

<210> 23
<211> 31
<212> DNA
<213> Artificial Sequence

<220>
<223> Description of Artificial Sequence:Synthetic DNA (primer)

<400> 23
cagcacccta gaaccaaatc cagcagactg c                                     31

<210> 24
<211> 31
<212> DNA
<213> Artificial Sequence

<220>
<223> Description of Artificial Sequence:Synthetic DNA (primer)

<220>
<221> misc_feature
<222> (31)... (31)
<223> n stands for any base

<400> 24
cagcaccctt gaaccaaatc cagcagactg n                                    31


<210> 25
<211> 33
<212> DNA
<213> Artificial Sequence

<220>
<223> Description of Artificial Sequence:Synthetic DNA (primer)

<400> 25
tcagaatcag ccaggcacaa agctgttgaa ttt                                  33


<210> 26
<211> 33
<212> DNA
<213> Artificial Sequence

<220>
<223> Description of Artificial Sequence:Synthetic DNA (primer)

<220>
<221> misc_feature
<222> (33)... (33)
<223> n stands for any base

<400> 26
tcagaatcag ccaggcacaa agcagttgaa ttn                                  33


<210> 27
<211> 25

<212> DNA
<213> Artificial Sequence

<220>
<223> Description of Artificial Sequence:Synthetic DNA (primer)

<400> 27
aaatctccct ccaaaagtgg tgctc                                                25


<210> 28
<211> 25
<212> DNA
<213> Artificial Sequence

<220>
<223> Description of Artificial Sequence:Synthetic DNA (primer)

<220>
<221> misc_feature
<222> (25)...(25)
<223> n stands for any base

<400> 28
aaatctccct ctaaaagtgg tgctn                                                25


<210> 29
<211> 30
<212> DNA
<213> Artificial Sequence

<220>
<223> Description of Artificial Sequence:Synthetic DNA (primer)

<400> 29
ctgaatataa acttgtggta gttggagctg                                           30


<210> 30

<211> 30
<212> DNA
<213> Artificial Sequence

<220>
<223> Description of Artificial Sequence:Synthetic DNA (primer)

<220>
<221> misc_feature
<222> (30)...(30)
<223> n stands for any base

<400> 30
ctgaatataa acttgtggta gttggaggtn                                        30


<210> 31
<211> 31
<212> DNA
<213> Artificial Sequence

<220>
<223> Description of Artificial Sequence:Synthetic DNA (primer)

<400> 31
ctgaatataa acttgtggta gttggagctg g                                      31


<210> 32
<211> 31
<212> DNA
<213> Artificial Sequence

<220>
<223> Description of Artificial Sequence:Synthetic DNA (primer)

<220>
<221> misc_feature
<222> (31)...(31)
<223> n stands for any base

<400> 32
ctgaatataa acttgtggta gttggaggtg n 31

<210> 33
<211> 23
<212> DNA
<213> Artificial Sequence

<220>
<223> Description of Artificial Sequence:Synthetic DNA (primer)

<400> 33
cttgtggtag ttggagctgg tgg 23

<210> 34
<211> 23
<212> DNA
<213> Artificial Sequence

<220>
<223> Description of Artificial Sequence:Synthetic DNA (primer)

<220>
<221> misc_feature
<222> (23)... (23)
<223> n stands for any base

<400> 34
cttgtggtag ttggaggtgg tgn 23

<210> 35
<211> 26
<212> DNA
<213> Artificial Sequence

<220>

<223> Description of Artificial Sequence:Synthetic DNA (primer)

<400> 35
gagcacatga cggaggttgt gaggcg                                          26

<210> 36
<211> 26
<212> DNA
<213> Artificial Sequence

<220>
<223> Description of Artificial Sequence:Synthetic DNA (primer)

<220>
<221> misc_feature
<222> (26)... (26)
<223> n stands for any base

<400> 36
gagcagatga cggaggttgt gaggcn                                          26

<210> 37
<211> 26
<212> DNA
<213> Artificial Sequence

<220>
<223> Description of Artificial Sequence:Synthetic DNA (primer)

<400> 37
gtatttggat gacagaaaca cttttc                                          26

<210> 38
<211> 26
<212> DNA
<213> Artificial Sequence

<220>
<223> Description of Artificial Sequence:Synthetic DNA (primer)

<220>
<221> misc_feature
<222> (26)... (26)
<223> n stands for any base

<400> 38
gtatttggat gacagacaca cttttn                                          26


<210> 39
<211> 30
<212> DNA
<213> Artificial Sequence

<220>
<223> Description of Artificial Sequence:Synthetic DNA (primer)

<400> 39
ggagtatttg gatgacagaa acacttttcg                                      30


<210> 40
<211> 30
<212> DNA
<213> Artificial Sequence

<220>
<223> Description of Artificial Sequence:Synthetic DNA (primer)

<220>
<221> misc_feature
<222> (30)... (30)
<223> n stands for any base

<400> 40
ggagtatttg gatgacagac acacttatcn                                      30

<210> 41
<211> 28
<212> DNA
<213> Artificial Sequence

<220>
<223> Description of Artificial Sequence:Synthetic DNA (primer)

<400> 41
   gtatttggat gacagaaaca cttttcga                                    28


<210> 42
<211> 28
<212> DNA
<213> Artificial Sequence

<220>
<223> Description of Artificial Sequence:Synthetic DNA (primer)

<220>
<221> misc_feature
<222> (28)...(28)
<223> n stands for any base

<400> 42
gtatttggat gacagacaca cttatcgn                                     28


<210> 43
<211> 27
<212> DNA
<213> Artificial Sequence

<220>
<223> Description of Artificial Sequence:Synthetic DNA (primer)

<400> 43
acatgtgtaa cagttcctgc atgggcg                                      27

<210> 44
<211> 27
<212> DNA
<213> Artificial Sequence

<220>
<223> Description of Artificial Sequence:Synthetic DNA (primer)

<220>
<221> misc_feature
<222> (27)... (27)
<223> n stands for any base

<400> 44
acatctgtaa cagttcctac atgggcn                                          27


<210> 45
<211> 28
<212> DNA
<213> Artificial Sequence

<220>
<223> Description of Artificial Sequence:Synthetic DNA (primer)

<400> 45
acatgtgtaa cagttcctgc atgggcgg                                         28


<210> 46
<211> 28
<212> DNA
<213> Artificial Sequence

<220>
<223> Description of Artificial Sequence:Synthetic DNA (primer)

<220>

<221> misc_feature
<222> (28)... (28)
<223> n stands for any base

<400> 46
acatctgtaa cagttccttc atgggcgn                                          28

<210> 47
<211> 32
<212> DNA
<213> Artificial Sequence

<220>
<223> Description of Artificial Sequence:Synthetic DNA (primer)

<400> 47
gtgtaacagt tcctgcatgg gcggcatgaa cc                                      32

<210> 48
<211> 32
<212> DNA
<213> Artificial Sequence

<220>
<223> Description of Artificial Sequence:Synthetic DNA (primer)

<220>
<221> misc_feature
<222> (32)... (32)
<223> n stands for any base

<400> 48
gtgtaacagt tcctgcttgg gcggcatgaa cn                                      32

<210> 49
<211> 26
<212> DNA

<213> Artificial Sequence

<220>
<223> Description of Artificial Sequence:Synthetic DNA (primer)

<400> 49
actgggacgg aacagctttg aggtgc                                          26


<210> 50
<211> 26
<212> DNA
<213> Artificial Sequence

<220>
<223> Description of Artificial Sequence:Synthetic DNA (primer)

<220>
<221> misc_feature
<222> (26)...(26)
<223> n stands for any base

<400> 50
actgggacgg aacaggtttg aggtgn                                          26


<210> 51
<211> 26
<212> DNA
<213> Artificial Sequence

<220>
<223> Description of Artificial Sequence:Synthetic DNA (primer)

<400> 51
ctgggacgga acagctttga ggtgcg                                          26


<210> 52
<211> 26

<212> DNA
<213> Artificial Sequence

<220>
<223> Description of Artificial Sequence:Synthetic DNA (primer)

<220>
<221> misc_feature
<222> (26)...(26)
<223> n stands for any base

<400> 52
ctgggacgga acaggtttga ggtgcn                                          26


<210> 53
<211> 33
<212> DNA
<213> Artificial Sequence

<220>
<223> Description of Artificial Sequence:Synthetic DNA (primer)

<400> 53
cctggctgat tctgaagata aactagaacc ctg                                  33


<210> 54
<211> 33
<212> DNA
<213> Artificial Sequence

<220>
<223> Description of Artificial Sequence:Synthetic DNA (primer)

<220>
<221> misc_feature
<222> (32)...(32)
<223> n stands for any base

<400> 54
cctggctgat actgaagata aactggaacc ctn                                33

<210> 55
<211> 29
<212> DNA
<213> Artificial Sequence

<220>
<223> Description of Artificial Sequence:Synthetic DNA (primer)

<400> 55
   acttttggag ggagatttcg ctcctgaag                                      29

<210> 56
<211> 29
<212> DNA
<213> Artificial Sequence

<220>
<223> Description of Artificial Sequence:Synthetic DNA (primer)

<220>
<221> misc_feature
<222> (29)... (29)
<223> n stands for any base

<400> 56
   acttttggag ggagatttcg ctgctgaan                                      29

<210> 57
<211> 33
<212> DNA
<213> Artificial Sequence

<220>
<223> Description of Artificial Sequence:Synthetic DNA (primer)

<400> 57
ggtctcctga acatagtgtt caggtggact ttt                                33

<210> 58
<211> 33
<212> DNA
<213> Artificial Sequence

<220>
<223> Description of Artificial Sequence:Synthetic DNA (primer)

<220>
<221> misc_feature
<222> (33)...(33)
<223> n stands for any base

<400> 58
ggtctccaga acatagtgtt caggtggact ttn                                33

<210> 59
<211> 29
<212> DNA
<213> Artificial Sequence

<220>
<223> Description of Artificial Sequence:Synthetic DNA (primer)

<400> 59
gtaggtgctt tatttttagg tacttctcg                                     29

<210> 60
<211> 29
<212> DNA
<213> Artificial Sequence

<220>

<223> Description of Artificial Sequence:Synthetic DNA (primer)

<220>
<221> misc_feature
<222> (29)...(29)
<223> n stands for any base

<400> 60
gtaggtgctt tatttttagg aacttctcn                                    29

## Description

The present application claims priority from Japanese application JP 2004-324138 filed on November 8, 2004, the content of which is hereby incorporated by reference into this application.

### BACKGROUND OF THE INVENTION

The present invention relates to a technique for detecting a gene mutation in a sample nucleic acid in a specimen. More specifically, it relates to a technique for detecting the presence or absence of any mutation in a sample nucleic acid having plural possible mutation sites, which is necessary for identification of a cancerous cell.

### FIELD OF THE INVENTION

The incidence of cancers increases with aging of the population. Stomach cancer, lung cancer and colon cancer are in descending order of incidence in males, and stomach cancer, breast cancer and colon cancer are in descending order of incidence in females. The incidence rate of stomach cancer decreases, but those of colon cancer and breast cancer significantly increase with each passing year with westernization of life habits. The survival rates of cancers have been improved from the viewpoint of treatment thereof with advancement of early detection including medical checkup and treatment methods. The recovery rate (cure rate) of stomach cancer, for example, was less than 40% twenty years ago, but is 50% or more in recent years. Likewise, the cure rates of colon cancer and breast cancer are 60% or more, and about 75%, respectively.

Abnormalities of the genes in cancers are roughly classified as three categories of oncogenes, antioncogenes and mutator genes. The oncogenes have been found based on the fact that causal genes in carcinogenic virus were found in human DNA sequences. Many of them transduce signals for allowing growth of cells. Alternatively, genes involved directly in DNA synthesis abnormally proliferate or deviate from normal control, to thereby induce carcinogenesis of cells. The antioncogenes were found as deleted genes in families suffering from cancers at a high rate. In contrast to the oncogenes, most of the antioncogenes inhibit the cell proliferation. Thus, the antioncogenes are compared to a brake, whereas the oncogenes are compared to an accelerator. More specifically, deletion or mutation of an antioncogene cannot stop the excursion of an oncogene and, as a result, invites carcinogenesis. When a mutator gene lacks its function, a mutation rate increases. These mutator genes serve to correct a miscopy occurred during replication of a DNA bearing genetic information in normal cell proliferation. If the mutator gene becomes abnormal, the frequency of miscopy of the DNA increases, which may lead to carcinogenesis. As is described above, certain genes or genetic abnormalities are involved in carcinogenesis. Utilizing gene mutation may possibly realize diagnosis of genes relating to cancers, part of which has been in practical use. This technique receives attention for clinical application to increasing ranges of areas.

Cancerous cells generally show abnormalities in DNA sequence. Many of such abnormalities occur regardless of the species of cancer, as in p53, ras family and myc gene family. In some species of cancers, however, the site of mutation varies in the same gene. DNA must be extracted from a diseased tissue and tested for detecting and monitoring these abnormalities of gene. The specimen for use herein includes, for example, one directly sampled from a diseased part, and peel-off cells recovered from sputum in the case of lung cancer, urine in the case of bladdery cancer, or stools in the case of colon cancer. The peel-off cells are contained in such specimens in a small amount. A body fluid is also an effective specimen, because cancerous cells left from a primary tumor cluster diffuse into the blood vessels, lymphatics and/or abdominal cavity and develop systemic metastases. In any case, the DNAs once extracted from the specimens are subjected to the same test procedure.

A bioluminometric assay coupled with modified primer extension reactions technique (BAMPER) (Guo-hua Zhou, et al., Nucleic Acid research, 29. e93 (2001)) is one of suitable procedures for analyzing nucleic acid mutation in respective specimens and has been investigated for practical use (Y Nakashima, et al., Clinical Chemistry, 50, 8 (2004)). The BAMPER is a technique for analyzing a base mutation using bioluminescence. In the BAMPER, a sample is subjected to an extension reaction with two to four different primers having A, G, C and T at the 3' end, respectively, and corresponding to types of mutation. The extension reaction proceeds only with a primer having a base type matching with that of a site to be detected. Pyrophosphate generated in the extension reaction is converted into ATP, and ATP is subjected to a luciferin-luciferase reaction to thereby allow light emission. The base can be typed within several minutes by detecting the light emission. This technique uses a simple reaction system to which a reagent is to be added and enables analysis of mutation using a luminescence measuring device having a simple optical system and is believed to be suitable for use in clinical sites and general laboratorics. This easy and convenient technique is also effective for detecting a point mutation generally observed in cancerous tissue cells, insertion or deletion of one to several bases, and single-nucleotide polymorphism (SNP).

Many of methods for analyzing gene mutation, as well as the BAMPER, generally use a sample extracted from the genome and a region containing a mutation site to be detected is amplified typically by a polymerase chain reaction. For example, the type of base mutation is determined from a product of PCR by DNA sequencing, or the base type is determined by carrying out a complementary strand extension (complementary strand synthesis reaction) using a DNA polymerase and a primer designed so as to have a sequence corresponding to the target mutated base at the 3' end and detecting the presence or absence of a product of the complementary strand synthesis (c.g., Japanese Patent No. 2853864). In detection of gene mutation as an application of the BAMPER, a complementary strand extension (complementary strand synthesis reaction) is carried out using a DNA polymerase and primers designed so as to have a sequence corresponding to the target at the 3' end, the resulting pyrophosphate is converted into ATP, and the presence or absence of extension is detected by using a bioluminescence reaction system with the use of a luciferase.

### SUMMARY OF THE INVENTION

A kit for detecting a mutation in a cancerous cell includes a reactor containing primers (oligodcoxyribonucleotides) for detecting mutation immobilized thereto and is so configured as to hybridize a sample DNA with the primer and to detect the presence or absence of a mutation by detecting a coloring reaction subsequent to a chemical reaction. The kit is so configured as to detect one base mutation per one reaction. In general, the combination of sites to which mutation is introduced in a target gene is often specified when the target of a specific cancer is specified. In this case, required information is whether or not mutation occurs at least one of the sites, and the information that on which site the mutation occurs is not so important. Accordingly, an object of the present invention is to provide a technique for easily and conveniently detecting whether or not "mutation occurs at least one point in a cancerous cell" by preparing a panel of a combination of target sites corresponding to each species of cancer or each species of gene, and detecting one or more gene mutations in a panel simultaneously..

Mutation-specific primers are designed and are subjected to reaction simultaneously under the same condition, in order to detect plural cancer-specific gene mutations efficiently in one reaction by using plural primers for detection of mutation. A panel of a combination of target sites corresponding to each species of cancer or each species of gene is prepared. The reaction efficiencies of the respective primers in the panel can be uniformized by controlling the concentrations of the primers.

Accordingly, the present invention provides, in an aspect, a method for analyzing a nucleic acid having plural possible mutation sites, including the steps of hybridizing plural primers with the nucleic acid, wherein the plural primers are complementary to any of the plural possible mutation sites at the 3' end, respectively, carrying out extension reaction of the hybridized plural primers, optically detecting a result of the extension reaction, and determining whether or not the nucleic acid has at least one mutation site based on the result in the step of optical detection, wherein the step of optical detection comprises determining the presence or absence of the extension by detecting light emission which is generated from at least one of the plural primers without distinction of species of the primers.

In the method, the step of optical detection may include carrying out a luminescence reaction with the use of a pyrophosphate generated in the step of carrying out extension to thereby detect a result of the extension as a result of the luminescence reaction. Inorganic pyrophosphate (PPi) is generated as a secondary-product in synthesis of a complementary DNA strand from a reaction substrate dNTP (deoxyribonucleotide triphosphate) in the presence (typically) of a DNA polymerase. The pyrophosphate is allowed to react with APS (adenosine 5'-phosphosulfate) in the presence of ATP sulfurylase to thereby form ATP. ATP acts as a substrate for luminescence reaction in the presence of luciferin and a luciferase to thereby emit light. The extension of the complementary strand can be detected by determining the emitted light.

The extension reaction may occur when the nucleic acid is complementary to at least two bases at the 3' end of any of the plural primers (mutation-specific primers).

The step of optical detection may include hybridizing plural probes with one or more products of the extension, and detecting fluorescence from a hybrid between any of the products and any of the probes, wherein the plural probes are labeled with a single-species fluorophore. The extension products can be labeled with a single-species fluorophore, and the step of optical detection can include detecting fluorescence with the emission wavelength of the single-species fluorophore.

In the step of hybridizing, the proportions of the primers may be changed. The proportions of the primers may be controlled so as to yield substantially the same emission intensity even when any of the plural primers is hybridized with the nucleic acid.

The present invention further provides, in another aspect, an apparatus for analyzing a nucleic acid, including a reactor for housing at least a sample nucleic acid and plural primers, a mechanism for controlling the temperature of the reactor, a detecting unit for detecting light emission in the reactor, and a control unit for analyzing the result detected by the detecting unit, wherein the detecting unit is so configured as to detect an extension between the sample nucleic acid and any of the plural primers as light emission without distinction of species of the primers and to determine the presence or absence of any mutation of the sample nucleic acid based on a result in the detection.

In addition and advantageously, the present invention provides, in yet another aspect, a kit for analyzing a nucleic acid, including plural primers which are complementary to any of plural possible mutation sites of a target nucleic acid at the 3' end, respectively, one or more enzymes for extending the primers, and a light-emitting reagent for optically detecting extension reaction of any of the primers, wherein the plural primers each have a sequence corresponding to any of the mutations. The primers may be at least one selected from primers listed in Table 1. The kit may be so configured as to prepare a panel of plural target sites on each target cancer or each target gene and to detect plural gene mutations in the panel simultaneously to thereby detect the presence or absence of mutation at least in one site in the sample nucleic acid. The primers may include at least one of the primers listed in Table 1 corresponding to mutations occurring in the K-ras gene, p53 gene or APC gene which are believed to be involved in cancers.

The above configuration can easily and conveniently detect the presence or absence of at least one mutation in one gene having plural possible mutation sites (sites where mutation can occur) in one reaction as the presence or absence of extension reaction. By using a fluorophore, the detection can be easily and conveniently carried out without the need of labeling probes corresponding to possible mutation sites with different fluorophores. To amplify plural sites simultaneously by a multiple polymerase chain reaction (PCR), the designing of a set of probes generally becomes complicated. In contrast, the extension in combination with a luminescence reaction according to the present invention does not so severely depend on the probe sequence as compared with PCR and can easily achieve simultaneous extension of plural sites. This is because the extension reaction for the luminescence detection is a linear reaction, whereas PCR is a non-linear reaction. Thus, by employing the luminescence reaction , a probe designed for a single site can be used as intact for detecting plural sites in one reaction, which realizes more simple and convenient detection.

The present invention can simultaneously detect one or more gene mutations in a panel of plural target sites on each species of cancer or gene in the analysis of gene mutation using an amplification or hybridization reaction of a nucleic acid segment with extension by a DNA polymerase and detection of light emission or fluorescence as a result of a subsequent light emission or color development reaction. This simplifies a test for detecting the presence of mutation in at least one site in a cancer cell, improves test efficiency and reduces time, effort and cost for the test.

Further objects, features and advantages of the present invention will become apparent from the following description of the preferred embodiments with reference to the attached drawings.

### BRIEF DESCRIPTION OF THE DRAWINGS

FIG. 1 shows a mutation site and the sequence therearound for illustrating the present invention;
FIG. 2 shows a mutation site for illustrating the present invention;
FIGS. 3-1, 3-2, 3-3, 3-4, 3-5 and 3-6 illustrate a mutation-specific extension of a primer for use in the present invention;
FIG. 4 is a structure of configurations of a primer and a spacer to be inserted into the primer for use in the present invention;
FIG. 5 illustrates a gene mutation to be detected according to the present invention;
FIG. 6 illustrates a mutation-specific extension of a primer for use in the present invention;
FIG. 7 is a diagram illustrating a mutant-specific extension of a primer mixture for use in the present invention;
FIGS. 8-1, 8-5, 8-6 and 8-7 show the relationship between proportions of primers in a primer mixture for use in the present invention and the extension of the primers;
FIGS. 9 and 10 show the results of mutation detection using a primer mixture according to the present invention;
FIG. 11 illustrates an analyzing apparatus according to the present invention; and
FIG. 12 illustrates primers for use in the present invention.

### DETAILED DESCRIPTION OF THE INVENTION

General reaction procedures for use in the examples of the present invention will be illustrated. A thermal cycler DNA Engine Tetrad (MJ RESEARCH) was used as a thermocontroller for extension in a reaction to synthesize a complementary strand in PCR using a DNA polymerase. The product of PCR was verified using a microchip electrophoresis analyzing system SV1210 (Hitachi High-Technologies Corporation). The syntheses of oligonucleotides were entrusted to Sigma-Genosys. The DNA polymerase was obtained from Amersham Biosciences, and other reagents were general commercially available products. The genome was purified from the blood provided by volunteers according to Molecular Cloning (Cold Spring Harbor Laboratory Press, Molecular Cloning (Second edition), 1989), unless otherwise specified.

PCR will be illustrated below as a representative example of extension. Each 1 µL of a 10 zmol/µL (1×10⁻²⁰ mol/µL) genomic DNA sample is placed in a 96-well PCR plate, and the plate is placed on ice. To each well are added 0.2 µL of 2.5 unit/µL Taq. DNA polymerase (QIAGEN), 4 µL of 2.5 mM dNTPs, and 0.8 µL each of a set of 25 pmol/µL primers. The volume is adjusted with sterile water to 100 µL per each well. The volumes of components can be changed while maintaining the same proportions. For example, PCR can be carried out on a 50 µL scale. The plate is sealed with an adhesive sheet and is placed in a thermal cycler. After heating at 94°C for two minutes for degenerating the genome, a thermal cycle of 94°C for 30 seconds, 57°C for 30 seconds, and 72°C for 1 minute is repeated a total of 35 times. The polymerase chain reaction mixture is analyzed by a microchip electrophoresis analyzing system to thereby determine the amount of the target PCR product. The microchip electrophoresis analyzing system for use herein employs an i-SDNA12 kit as a reagent kit. The system can automatically determine the length and amount of the target PCR product based on the length and amount of a base as an internal standard marker within an analysis range of 10 to 500 base pairs (bp). A total of 1 µL of the polymerase chain reaction product is analyzed in each example.

PCR using a thermal cycler has been illustrated as an example. The synthesis of one strand of a target genome or DNA using either one primer alone can also be carried out, and the amplification product can be amplified by carrying out a cyclic reaction in the same manner as in PCR. Alternatively, the extension reaction can be carried out isothermally (e.g., at a constant temperature of 37°C) using an enzyme such as E. coli DNA polymerase I or its partial enzyme, i.e., Klenow fragment.

### EXAMPLES

The present invention will be illustrated in further detail with reference to several examples below, which are never intended to limit the scope of the present invention.

### Example 1

The morbidity of colon cancer has increased in recent years and a demand has been made to develop a more specific screening method, since the conventional immunological fecal occult blood test frequently shows false positive results. Some features of the present invention will be illustrated below by taking K-ras gene as an example, since mutation in the K-ras gene is observed in 50% of patients with colon cancer. The K-ras gene is a general tumor marker and shows mutation in many cancerous cells such as of lung cancer, stomach cancer, and liver cancer, in addition to colon cancer.

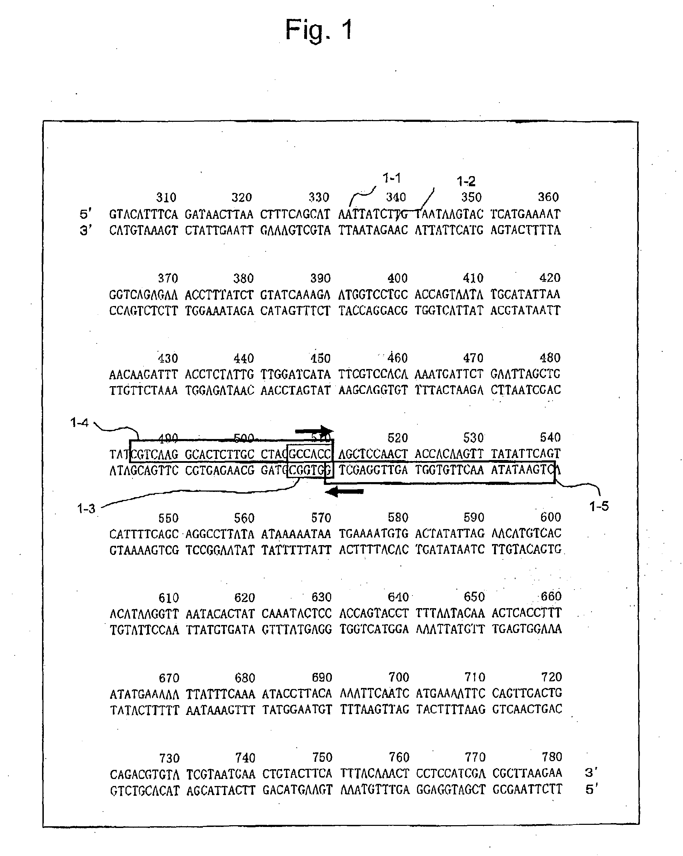

A method for determining or detecting base mutation using a complementary strand synthesis reaction as a representative of the extension will be illustrated with reference to FIGS. 1 to 3. FIG. 1 shows sample complementary double-stranded genome 1-1 and 1-2. This sequence information is available as Accession Number NT_009714 from NCBI, [online], [searched on September 27, 2004], internet, <URL: http://www.ncbi.nlm.nih.gov>. A total of 577 base pairs of the sequence 18157098 to 18157675 in NT_009714 is amplified according to the above-mentioned method. The resulting PCR product includes 6 bases of codon 12 and codon 1 (1-3 in FIG. 1) at a center part thereof. The PCR product is then purified by gel filtration using Sephadex G100 to remove primers and dNTPs used in the polymerase chain reaction. Alternatively, the target PCR product is purified by enzymatic cleaning up in the following manner. Specifically, 0.7 µL of 1 unit/µL shrimp alkaline phosphatase, 0.06 µL of 10 unit/µL exonuclease I, 0.3 µL 10× PCR buffer (Amersham Pharmacia) and 3.94 µL of sterile water are added to 50 µL of the reaction mixture after the polymerase chain reaction. The mixture is subjected to an enzymatic reaction with incubation at 37°C for 40 minutes, followed by inactivation of the enzyme by heating at 80°C for 15 minutes. This substantially purifies the target PCR product. Gel filtration using Sephadex G25 or G50 before the enzymatic cleaning up can purify the target PCR product to a higher purity.

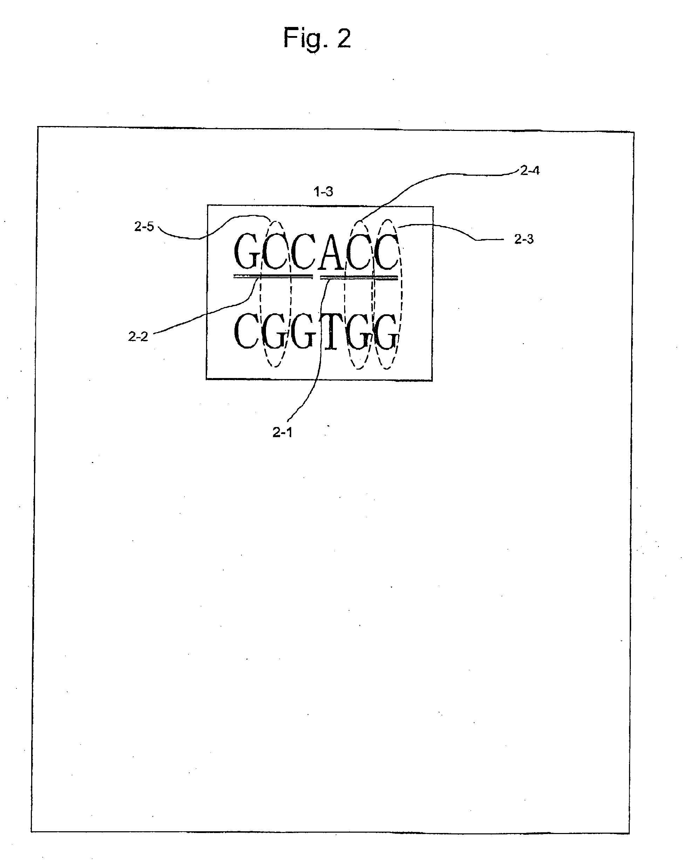

The region 1-3 in the purified PCR product comprising a complementary double strand includes codon 12 (the region 2-1 in FIG. 2) and codon 13 (the region 2-2 in FIG. 2). A C to A mutation (2-3) and a C to A or T mutation (2-4) may occur in codon 12. A C to T mutation (2-5) may occur in codon 13.

Upper side and lower side primer designs (1-4 and 1-5) for the determination of the region 2-3 (K-ras codon 12(A)) are shown in FIG. 1. The base type of the region 2-3 is determined by hybridizing the primer 1-4 or 1-5 with the template DNA strand 1-2 or 1-1, followed by extension. The primer is so designed as to have its 3' end at the point 2-3. A complementary strand synthesis reaction by the action of a DNA polymerase occurs if the 3' end of the primer is complementary to the base 2-3 of the template DNA strand 1-1 or 1-2 of the PCR product. If not, the complementary strand synthesis reaction does not occur or occurs only to a small extent. When the base 2-3 is C, for example, the complementary strand synthesis occurs only if the 3' end of the primer 1-4 is G. The complementary strand synthesis reaction hardly occurs when the 3' end is C, A or T. Thus, the base 2-3 can be typed by determining whether or not the extension occurs using primers having G, C, A and T at the 3' end, respectively.

Whether or not the extension occurs can be determined by converting pyrophosphate generated during the complementary strand synthesis into ATP by the action of an enzyme PPDK (pyruvate orthophosphate dikinase) and determining the amount of ATP using a luciferin-luciferase system. If the complementary strand synthesis reaction occurs, one molecule of pyrophosphate is generated as a result of extension of one base. Accordingly, 442 molecules of pyrophosphate are generated as a result of hybridization of the primer with the PCR product and extension to the end thereof. The molecules of pyrophosphate are then converted ATPs, and ATPs induce light emission in the luciferin-luciferase system. If the complementary strand synthesis does not occur and pyrophosphate is not generated, and the luciferin-luciferase system does not emit light. However, some light emission may occur even in this case, because of non-specific reaction of the primer and/or degradation of the substrate dNTP.

Specific procedures for detecting the extension by using a luminescence reaction in the luciferin-luciferase system will be described below. The reaction mixture of the polymerase chain reaction is, for example, subjected to enzymatic cleaning up in the same way as above and is cooled to 4°C. Each 2 µL of the reaction mixture is divided into each well of a 96-well PCR plate (white). A total of 1 µL of the primer 1-6 (5 pmoL/µL) is added thereto. In addition, 0.0275 µL of 5 unit/µL Taq. DNA polymerase and 0.04 µL of 5 mM dNTPs are added to sterile water up to 1.0µL. Each 1.0µL of the resulting solution is added to each well. A mineral oil (4 µL) is placed thereon. A cycle of 94°C for 10 seconds and 55°C for 10 seconds is repeated a total of five times, followed by cooling to 25°C. Each 10 µL of a light-emitting reagent previously held to 25°C is added, the resulting mixture is stirred by pipetting; and the light emission is determined using a luminometer. The light-emitting reagent is a bioluminescence kit for converting pyrophosphate into ATP and detecting ATP by luciferin-luciferase system. This procedure easily detects whether or not the extension occurs based on the emission intensity depending on the amount of pyrophosphate.

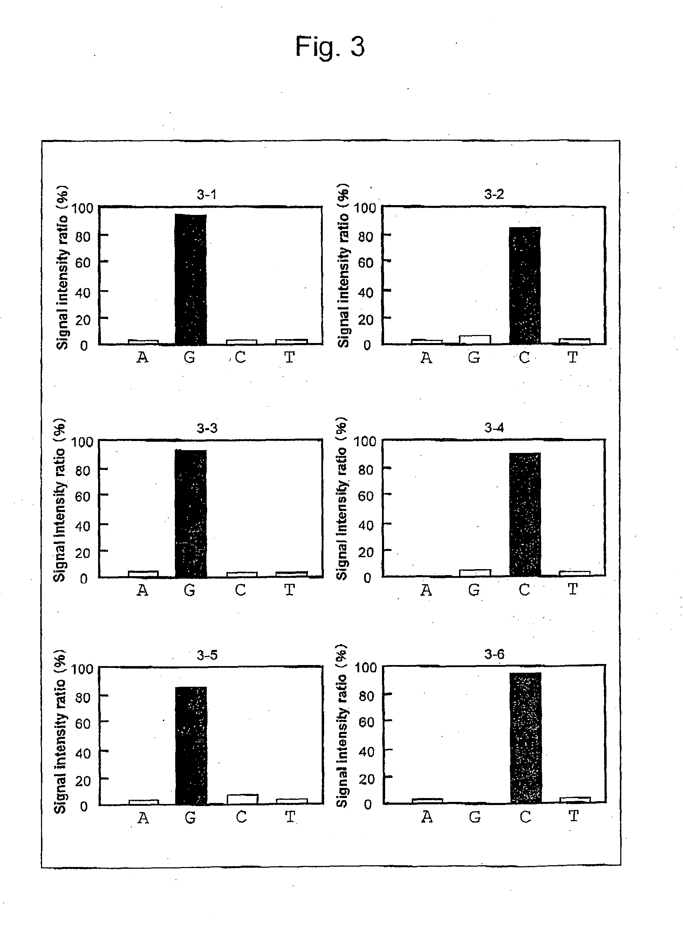

Detection of the mutations 2-3, 2-4 and 2-5 are shown in FIGS. 3-1, 3-2, 3-3, 3-4, 3-5 and 3-6. Primers having four different bases at the 3' end, respectively, are allowed to react with a PCR product derived from DNA of a cultured cell (as alternate of a sample of healthy control) which had been verified to have no mutation. FIGS. 3-1 and 3-2 illustrate detection (typing) of the base 2-3 in FIG. 2, which base 2-3 is C in a healthy control. When forward primers (1-4 in FIG. 1) are used for the detection of the base 2-3, the extension occurs only when a primer having G at the 3' end is used, and the extension is detected in terms of the emission intensity (FIG. 3-1). Primers having A, C or T at the 3' end do not invite extension, and the extension in terms of light emission is not detected. When reverse primers (1-5 in FIG. 1) are used for the detection of the base 2-3, the extension occurs only when a primer having C at the 3' end is used, and the extension is detected in terms of the emission intensity (FIG. 3-2). Primers having A, G or T at the 3' end do not invite extension, and the extension in terms of light emission is not detected. Likewise, FXGS. 3-3 and 3-4 illustrate detection (typing) of the base 2-4 in FIG. 2, which base 2-4 is C in a healthy control. When forward primers are used, the extension occurs only when a primer having G at the 3' end is used, and the extension is detected in terms of the emission intensity (FIG. 3-3). When reverse primers are used, the extension occurs only when a primer having C at the 3' end is used, and the extension is detected in terms of the emission intensity (FIG. 3-4). FIGS. 3-5 and 3-6 illustrate detection (typing) of the base 2-5 in FIG. 2, which base 2-5 is C in a healthy control. When forward primers are used, the extension occurs only when a primer having G at the 3' end is used, and the extension is detected in terms of the emission intensity (FIG. 3-5). When reverse primers are used, the extension occurs only when a primer having C at the 3' end is used, and the extension is detected in terms of the emission intensity (FIG. 3-6). Thus, the bases can be typed exactly.

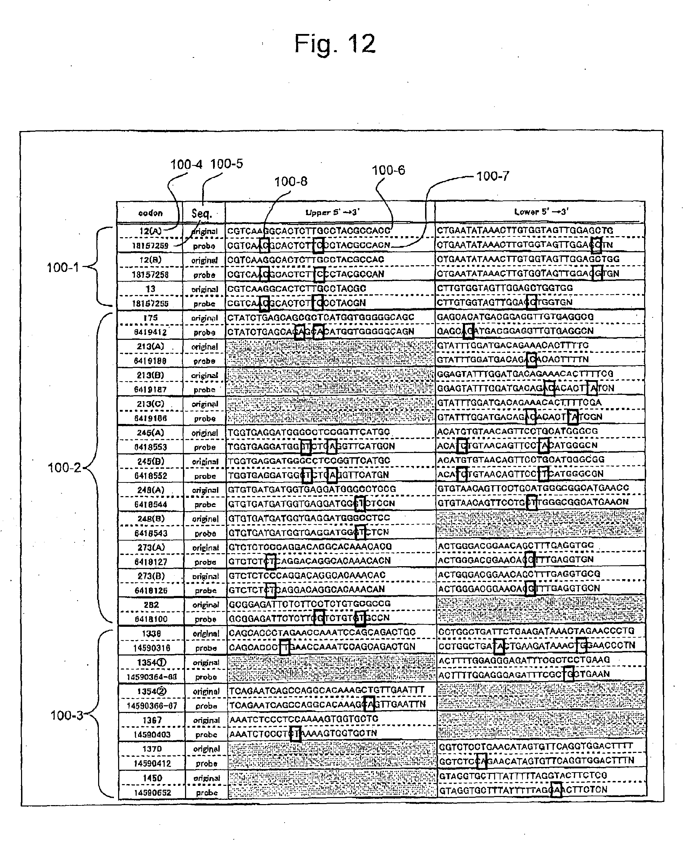

Primers having designed mismatches for analyzing gene mutations in the K-ras, p53 and APC genes believed to generally occur in colon cancer are shown in Table 1 and FIG. 12. FIG. 12 is for the sake of explanation. Table 1 and FIG. 12 show the K-ras gene (NCBI Accession Number: NT_009714.16) 100-1, the p53 gene (NCBI Accession Number: NT_010718.14) 100-2, and the APC gene (NCBI Accession Number: NT_034772.5) 100-3. Figures represented by the reference number 100-4 are the numbers of codon of the gene on which a mutation occurs. If two or more mutations occur in one codon, the mutations are represented by (A), (B), (C)... from the front of the triplet. If there are two or more patterns in the mutation occurring in one codon, the patterns are represented by (i), (ii), (iii)... The figures represented by the reference numeral 100-5 are the point on the base sequence shown in the NCBI Accession Number. An original sequence 100-6 around a target mutation site, and a sequence 100-7 of a primer having a mismatch used in the analysis are also shown. In the sequence just mentioned above, the base N at the 3' end is any one of the four bases, A, G, C and T. Each of the primers essentially contains any of mismatches 100-8.

The mismatch was inserted so as not to have a structure in which a synthetic oligodeoxyribonucleotide for a primer invites intramolecular hybridization on three or more successive bases in the molecule and invites holding. More specifically, in the primer, one or two bases of the three successive bases relating to the holding in the synthetic oligodeoxyribonucleotide are replaced with spacers or bases not complementary to the template DNA. In addition, the site where the bases are replaced with spacers or non-complementary bases are positioned three bases or more far from the 3' end of the primer.

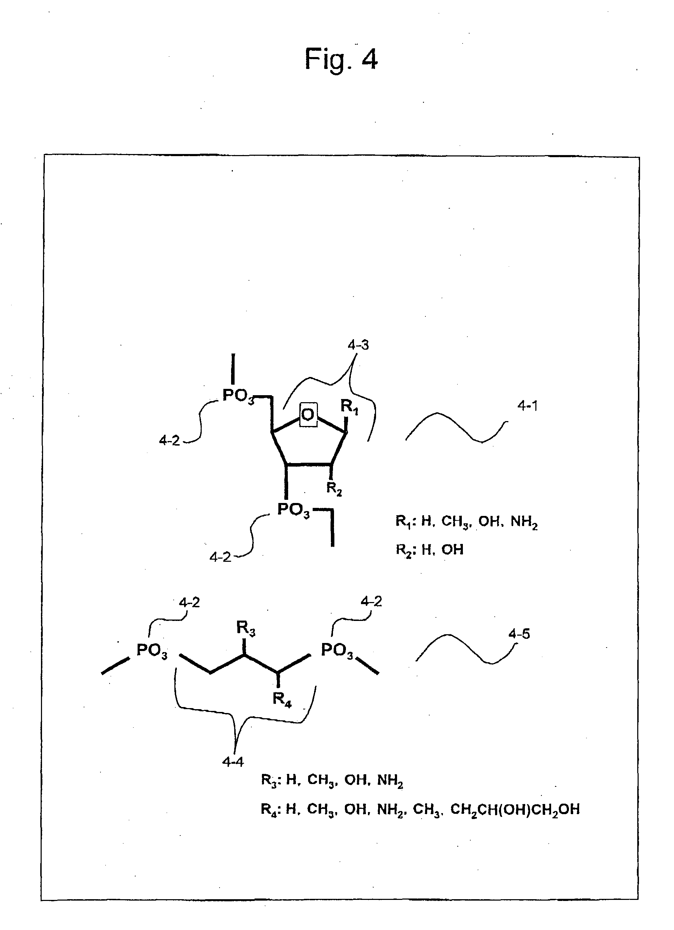

A spacer having a structure 4-1 wherein R₁ and R₂ are H (hydrogen) and/or one having a structure 4-5 wherein R₃ and R₄ are H shown in FIG. 4 can be used. The length of the primer is not limited to those shown in Table 1, since the length can be ten bases or more up to fifty bases as counted from the target base at the 3' end. The length of the primer can be arbitrarily set according to the reaction type (solid phase reaction or liquid phase reaction), the volume of the reaction mixture, and the reaction efficiency due to the shape of the reactor. When a test was carried out according to the above procedure, the primer length shown in Table 1 was optimal, and target mutated bases could be detected in use of any of the sequences. In this example, amplification by PCR is taken as an embodiment of the present invention, but the present invention can also be applied to any amplification in which a primer is hybridized with a priming site, and a complementary strand synthesis reaction is carried out using a polymerase, as in nucleic acid sequence-based amplification (NASBA), rolling-circle amplification and allele-specific polymerase chain reaction (ASP-PCR).

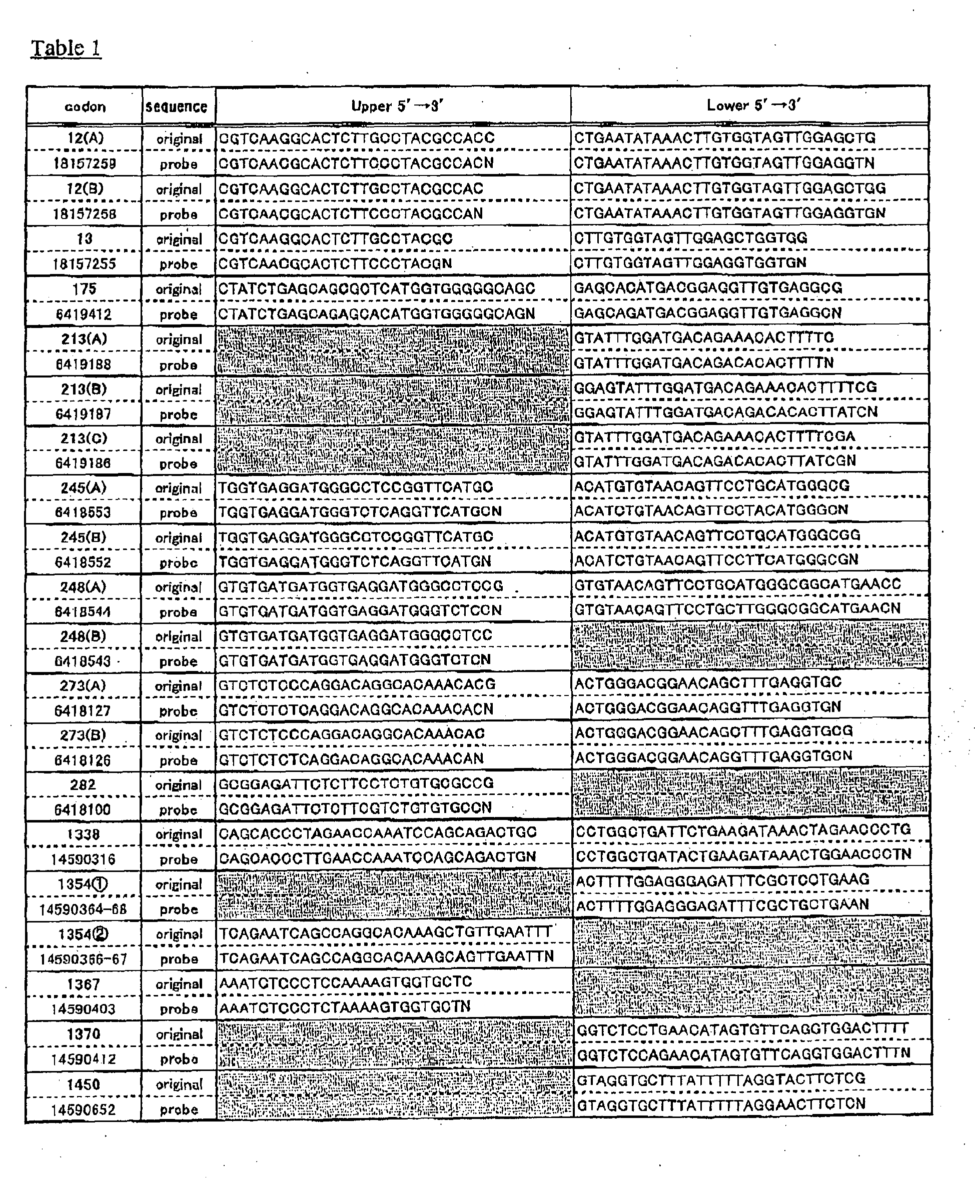

### Example 2

In actual, it is difficult to recover pure cancerous cells alone from a cancerous tissue or a body fluid such as sputum, urine stools, blood or faeces and extract DNA therefrom for analyzing gene mutation. Accordingly, primers for use in detection or analysis of mutation must be sufficiently specific so as not to react with normal cells.

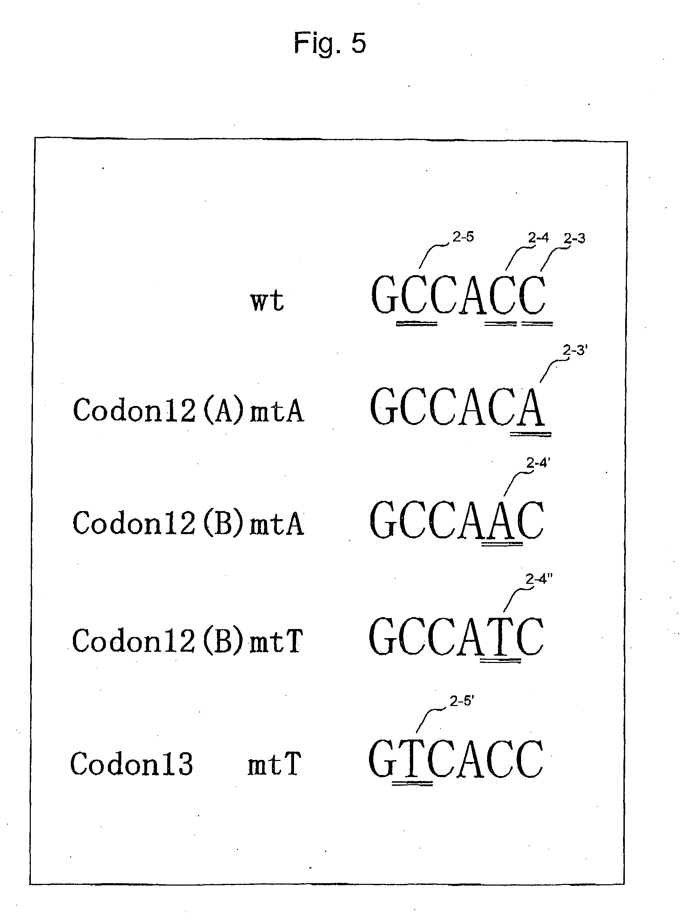

Mutations on the K-ras gene in case of colon cancer often occur in the codon 12 and the codon 13, and there are five patterns, including a wild type, of mutations on two codons and six bases as shown in FIG. 5. With respect to the normal base sequence wt, a C to A mutation (2-3') at the base 2-3 is referred to as codon "12(A) mtA". Likewise, a C to A mutation (2-4') at the base 2-4, a C to T mutation (2-4") at the base 2-4, and a C to T mutation (2-5') at the base 2-5 are referred to as "codon 12(B) mtA", "codon 12(B) mtT" and "codon 13 mtT", respectively.

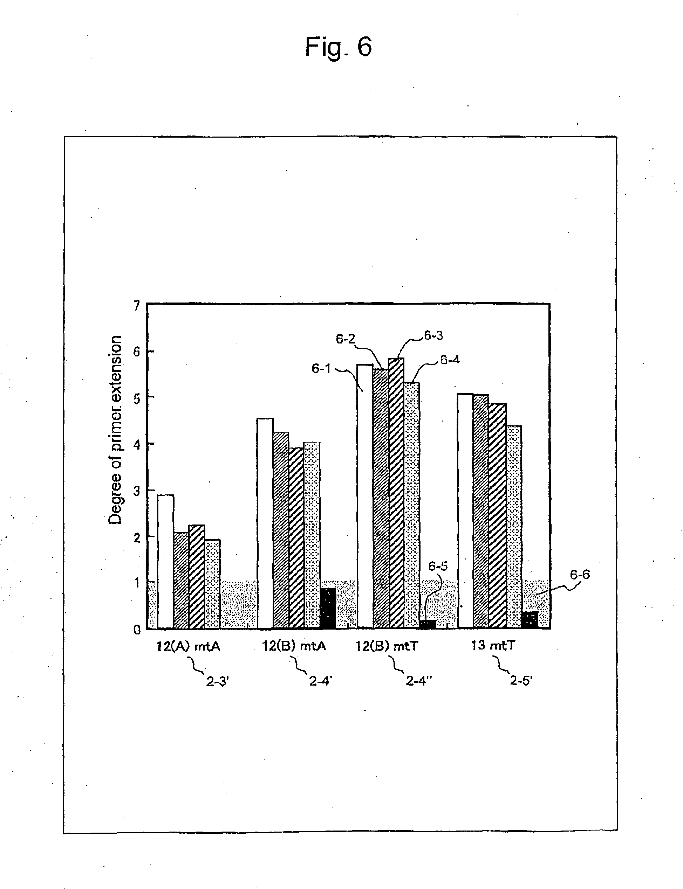

A mixture of these in arbitrary proportions was used as a model for test. Four primers corresponding to the mutations 2-3', 2-4', 2-4" and 2-5', respectively, were used in the determination, so as not to detect bases derived form a normal cell contained in a sample DNA. With reference to FIG. 6, five different samples were prepared for each mutation type (ml). The five samples include a sample 6-1 containing a base sequence corresponding to each mutation type (mt) alone, a sample 6-5 containing a base sequence of the wild type (wt) alone, a sample 6-2 containing 75% of the base sequence corresponding to each mutation type (mt) and 25% of the base sequence corresponding to the wild type (wt), a sample 6-3 containing 50% of the base sequence corresponding to each mutation type (mt) and 50% of the base sequence corresponding to the wild type (wt), and a sample 6-4 containing 25% of the base sequence corresponding to each mutation type (mt) and 75 % of the base sequence corresponding to the wild type (wt). The mutations were typed by allowing these samples to react with four primers having a base at the 3' end corresponding to the mutations 2-3', 2-4', 2-4" and 2-5', respectively.

In the analysis result, the degree of extension was defined as the value of emission intensity due to extension of the primer, provided that the emission intensity (background emission) where no primer was added to a sample was set at 1. The region 6-6 is a region where the emission intensity was less than two times the background emission, which region is below the detection limit in the detection according to BAMPER. Four different primers corresponding to each mutation (mt) were allowed to react with Samples 6-1, 6-2, 6-3, 6-4 and 6-5 containing 100%, 75 %, 50%, 25% and 0 % of the mutated base sequence. As a result, each mutation could be detected, and the wild type (wt) was not detected. Each of the primers was prepared as to have a final concentration of 5 pmol per reaction.

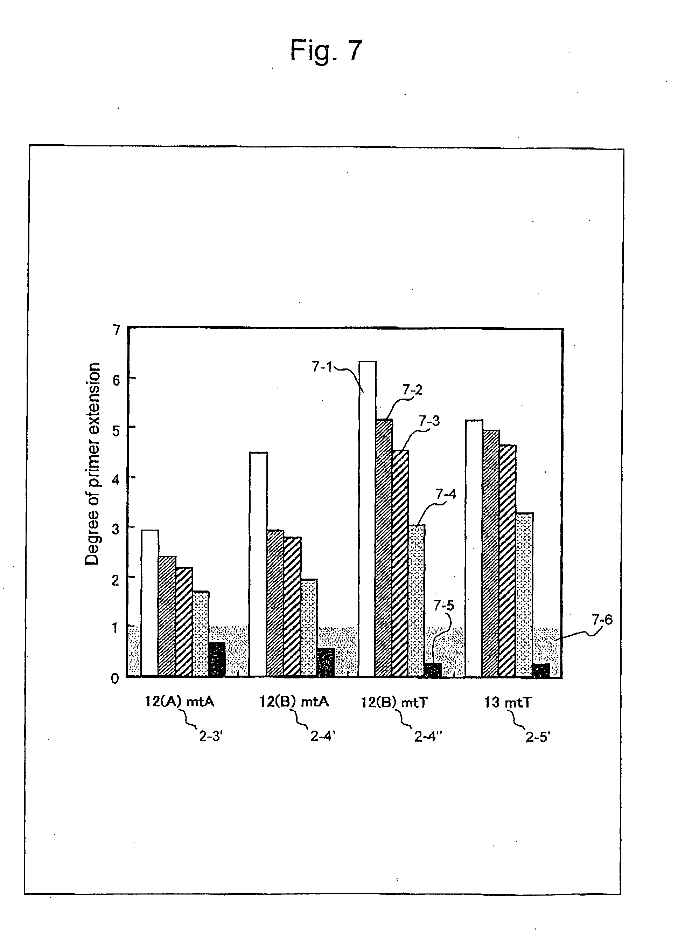

FIG. 7 shows the detection using a mixture of the four primers for mutation detection having a final concentration of 5 pmol per reaction. Samples 7-1, 7-2, 7-3, 7-4 and 7-5 contain 100%, 75%, 50%, 25% and 0% of the mutated base sequence, as in the test shown in FIG. 6. The region 7-6 is a region below the detection limit. The primer having the base type 2-3' at the 3' end for detecting the codon 12(A) mtA could detect the codon 12(A) mtA alone without being inhibited by the action of the other three primers. The mixture of four primers could also exactly detect the mutations codon 12(B) mtA, codon 12(B) mtT and codon 13 mtT as a result of target-specific reaction.

For amplifying multiple sites simultaneously according to multiple (quantitative) PCR, it takes much time and effort to design a primer set, and arbitrary multiple sites cannot always be amplified simultaneously. In contrast, the conditions for extension according to BAMPER less vary depending on the primer sequence, concentration and/or temperature than PCR and can easily achieve extension of multiple sites simultaneously. This is because BAMPER is a linear reaction, whereas PCR is a non-linear reaction. More specifically, a primer designed for detecting one single mutation site can also be used for detecting plural sites simultaneously according to BAMPER, in contrast, such a primer designed for detecting one single mutation site cannot be used for detecting plural mutation sites, and the primer must be newly designed according to PCR. These advantages of BAMPER are exhibited markedly in simultaneous reactions at plural sites and are features of BAMPER for use in detection of multiple sites simultaneously.

### Example 3

In the detection of four mutation sites shown in Example 2 (FIG. 6 and FIG. 7), the four primers for mutation detection have different reactivities. The primer having the base type 2-4" at the 3' end and corresponding to the codon 12(B) mtT has the highest reactivity, and the primer having the base type 2-3' at the 3' end and corresponding to the codon 12(A) mtA has the lowest reactivity. When a specific mutation on a specific gene is to be detected, the primers should be designed under a condition within a very narrow range. A slight deference in the base sequence of the primers (difference in one base type or length) affects the efficiency of hybridization with the template DNA (PCR product), resulting in a difference in extension efficiency. It is very difficult to set optimal annealing temperatures for the respective primers in detection of plural sites simultaneously, and the condition including annealing temperature has to be such a condition as to ensure the reaction efficiencies of all the primers used (four primers in the example) at certain level. However, the apparent signal intensity can be arbitrary controlled by changing the proportions of primers in a mixture in a linear reaction as in BAMPER, in contrast to a non-linear reaction as in the polymerase chain reaction.

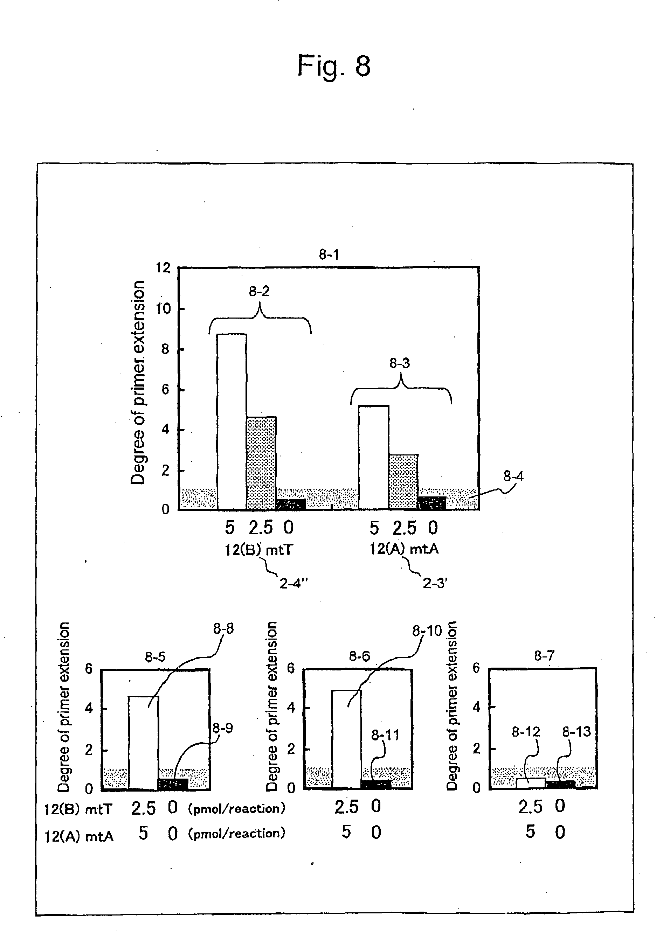

FIG. 8-1 shows the relationship between the emission intensity and the primer concentration. When a sample (100%) showing the codon 12(B) mtT was allowed to react with a primer for detection of the base type 2-4" at a concentration of 5 pmol/reaction or 2.5 pmol/reaction, the emission intensity decreased with a decreasing concentration of the primer (8-2). The same tendency was observed when a sample (100%) showing the codon 12(A) mtA was allowed to react with a primer for detection of the base type 2-3' at a concentration of 5 pmol/reaction or 2.5 pmol/reaction. The region 8-4 is a region where the emission intensity was less than two times the background emission, which region is below the detection limit in the detection according to BAMPER. The degree of extension of the primer for detecting the base type 2-4" at a concentration of 2.5 pmol/reaction in the test 8-2 was substantially equal to that of the primer for detecting the base type 2-3' at a concentration of 5 pmol/reaction in the test 8-3. Accordingly, the ratio of the concentration of the primer for the base type 2-4" to that of the primer for the base type 2-3' was set at 1:2 (2.5 pmol/reaction and 5 pmol/reaction) in a system for detecting mutations 2-4" and 2-3' simultaneously.

FIGS. 8-5, 8-6 and 8-7 show the results of tests using culture cells in which the presence or absence of mutation at a target point had been determined. With reference to FIG. 8-5, a mixture of the two primers was allowed to react with a specimen derived from a culture cell (as a patient model) having the codon 12(B) mtT mutation. As a result, only the primer 2-4" corresponding to the codon 12(B) mtT underwent reaction (extension) (8-8), and a control containing no primer did not undergo extension (8-9). With reference to FIG. 8-6, a mixture of the two primers was allowed to react with a specimen derived from a culture cell (as a patient model) having the codon 12(A) mtA mutation. As a result, only the primer 2-3' corresponding to the codon 12(A) mtA underwent reaction (extension) (8-10), and a control containing no primer did not undergo extension (8-11).

With reference to FIG. 8-7, a mixture of the two primers was allowed to react with a specimen derived from a culture cell having no mutation as a model of healthy control, the two primers did not undergo reaction (8-12) as in the control (8-13).

When a mixture of two or more different primers for mutation detection is allowed to react with a specimen, the proportions of the primers in the mixture should be preferably controlled so as to obtain the efficiencies of the respective primers at substantially equal level. Thus, the emission intensity (degree of primer extension) varies depending only on the amount of DNA in the specimen DNA or the amount of PCR amplified product. When the primer mixture is allowed to react with an unknown sample, a primer extension, if occurs, means the sample has mutation at a site corresponding to any of the primers in the primer mixture. In contrast, no primer extension indicates that the sample has no mutation corresponding to any of the primers in the primer mixture or that the sampled specimen contains a too small amount of cancerous cells to be detected. This configuration can minimize the difference in reaction efficiency (reactivity) among the primers. If there is such a difference in reactivity, a mutation corresponding to a primer having a high reactivity is highly possibly detected, but a mutation corresponding to a primer having a low reactivity is hardly detected.

These advantages can also be obtained even if the number of types of primers for mutation detection used in a panel of plural target sites varying upon each type of cancer or gene and can be considered as featured advantages of BAMPER for detecting multiple sites simultaneously. The present invention can be applied to a shifted termination assay (STA) and other techniques in which a color reaction occurred due to the presence or absence of extension using sequence-specific primers. It can be applied to a technique in which a target to be detected is labeled with DNP, is allowed to react with an anti-DNP-alkaline phosphatase antibody, and a color reaction as a result of the reaction between the alkaline phosphatase and the color-developing substrate is detected. It can be applied to a technique disclosed in Japanese Unexamined Patent Application Publication No. 2003-174900, in which extension using a mutation-specific primer is carried out as in BAMPER, pyrophosphate generated as a result of the extension is converted via a general chemical reaction into, for example, formazan, hydrogen peroxide, superoxide, carbon dioxide, L-phenylalanine or sulfate ion, and the resulting substance is detected by using a detector or by visual observation. It can also be applied to a technique in which extension using a mutation-specific primer is carried out, pyrophosphate generated as a result of the extension serves to be coupled with a metal ion of a fluorophore so as to deprive the metal ion from the fluorophore, the resulting fluorophore emits light because the metal ion has played a role as a quencher therein, and the amount of the fluorophore emitting light is detected by using a detector or by visual observation.

### Example 4

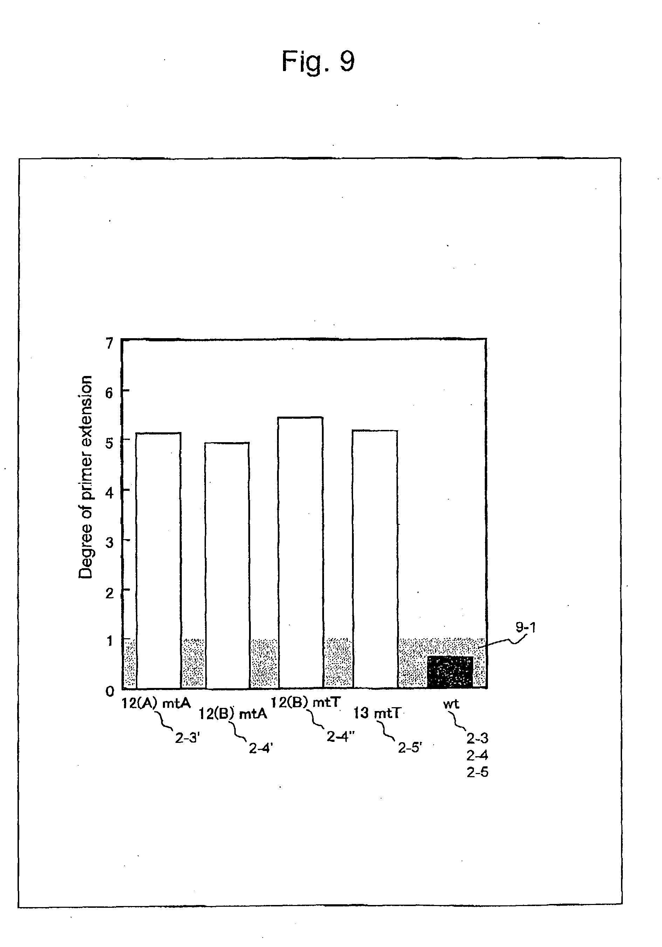

In the above examples, the presence or absence of extension of mutation-specific primers was detected using a luminescence reaction using pyrophosphate generated during extension. The extension can also be detected by using fluorescence. The designing of primers, conditions for hybridization reaction and extension are the same as above. In this example, plural probes capable of hybridizing with plural possible extension products and labeled with a single-species fluorophore were added after the extension reaction and were allowed to react with the extension products in a hybridization reaction. As the fluorophore, Cy3 having an emission wavelength of 570 nm was used. The reaction mixture containing the resulting hybrids was passed through a gel filtration column to remove unuceacted primers, the hybrids in the recovered mixture were excited by YAG laser light having a wavelength of 534.5 nm, and the presence or absence of extension was determined based on the quantity of emitted fluorescence. The results are shown in FIG. 9. Samples known to have the mutations 2-3', 2-4', 2-4" and 2-5', respectively, showed fluorescence emission at equal to or higher level than the standard, and a sample known to have no mutation showed fluorescence emission at a level equal to that of the background. The region 9-1 is a region below the detection limit. The results show that the presence or absence of extension of mutation-specific primers can also be detected by hybridization of fluorescence-labeled primers. A suitable fluorophore can be arbitrarily selected from among a variety of commercially available fluorophores according to the properties of a measuring apparatus to be used.

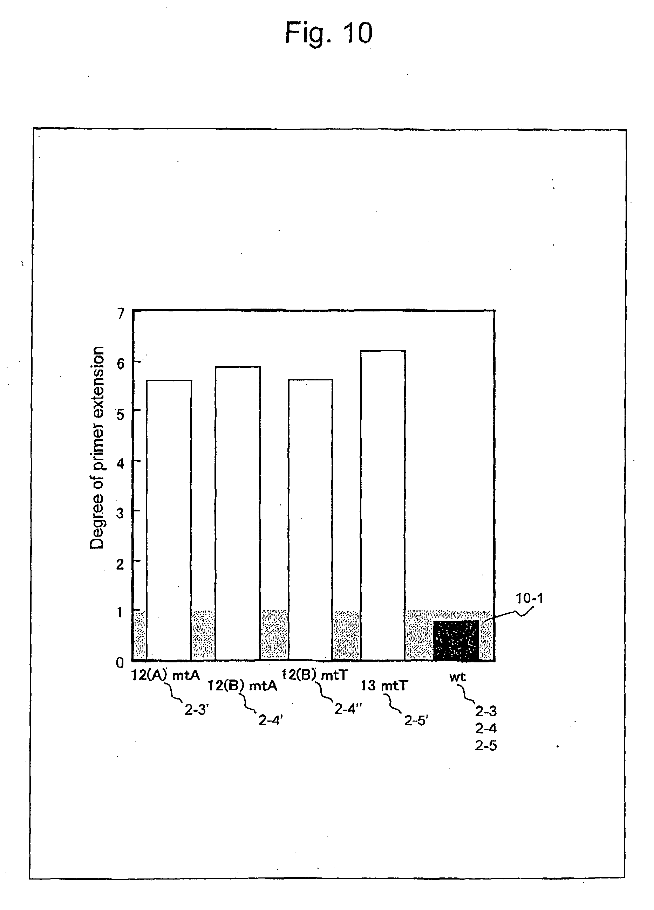

The same advantages can be obtained by allowing, instead of a primer labeled with a fluorophore, an intercalator to react with a sample, and detecting the resulting fluorescence. The intercalator is a substance capable of emitting fluorescence when intercalated into a double-stranded nucleic acid. The intercalator, if used, may be added to a reaction mixture for the complementary strand extension. The intercalator includes, for example, YOYO and Cyber Green. FIG. 10 illustrates a test using YOYO. Samples known to have the mutations 2-3', 2-4', 2-4" and 2-5', respectively, showed fluorescence emission at equal to or higher level than the standard, and a sample known to have no mutation showed fluorescence emission at a level equal to that of the background. The region 10-1 is a region below the detection limit.

This example shows that the presence or absence of extension of mutation-specific primers upon plural possible mutation sites can be detected by the action of fluorescence. A suitable intercalator can be arbitrarily selected according to the properties of a measuring apparatus to be used, from among a variety of commercially available intercalators.

### Example 5

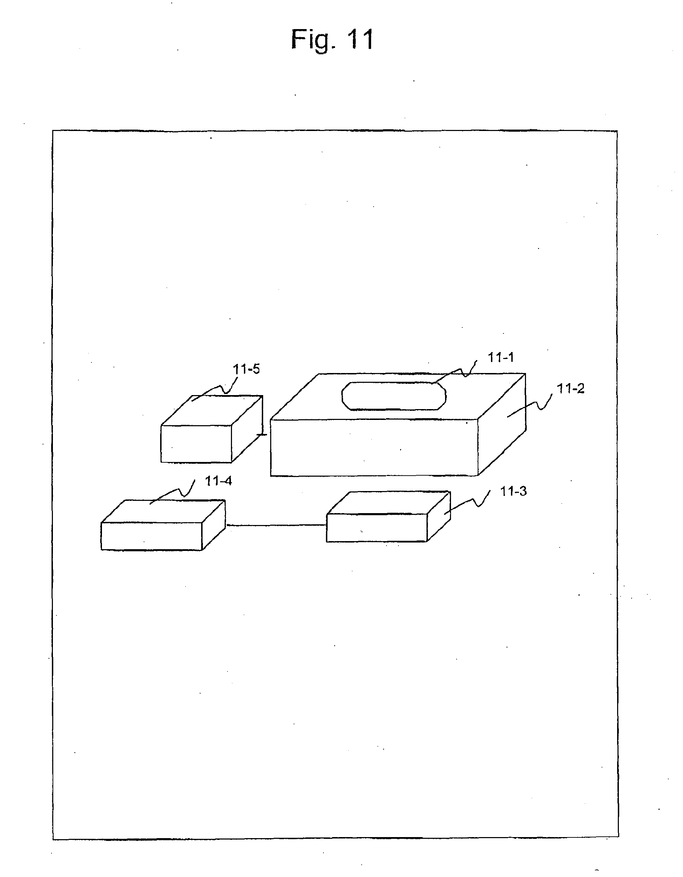

FIG. 11 illustrates a configuration of an apparatus which is so configured as to detect the presence or absence of at least one mutation site in a sample nucleic acid having possible mutation sites. The sample nucleic acid obtained from a specimen is housed with other components such as primers in a reactor 11-1. The sample nucleic acid undergoes hybridization with primers so as to form hybrids in the reactor 11-1. The primers are complementary to any of the plural possible mutation sites at the 3' end. The reactor 11-1 is housed in a reaction unit 11-2, and the temperature of the reactor 11-1 is controlled by a mechanism 11-5 for controlling the temperature. The hybridized primers undergo extension in the reactor under the temperature control of the mechanism 11-5 for controlling the temperature. In this procedure, the complementary strand extension proceeds when the 3' end of each primer is completely to the sample nucleic acid.

The extension produces pyrophosphate, which in turn reacts with a light-emitting reagent to emit bioluminescence, and the bioluminescence is detected by a detecting unit 11-3 having an optical system. The detecting unit may be arranged at the top or bottom of the reactor. In this procedure, light emission without distinction of species of the primers is detected.

The detection result in the detecting unit 11-3 is transmitted to a control unit 11-4. The control unit 11-4 determines whether or not the light emission is equal to or higher than the standard level, thereby detects the presence or absence of the extension, i.e., the presence or absence of any mutation in the possible sites to thereby determine whether or not the sample nucleic acid has any mutation site. This configuration realizes an analyzer capable of detecting whether or not a sample nucleic acid having plural possible mutation sites has any mutation site.

Gene alterations occurring in affected areas due to morbidity and progress thereof, such as point mutations, have been increasingly analyzed in the field of medicine. The relationship between the severity or progress of a disease and a variation of a specific base at a specific site in a specific gene has been revealed with increasing information. For efficiently carrying out medical checkups using the information, all the possible bases exhibiting mutation must be examined so as to combine the information on the presence or absence of mutation with information on the presence or absence of the possibility of cancer. BAMPER for detecting plural sites simultaneously according to the present invention simplifies a test for detecting the presence of mutation in at least one site in a cancer cell, improves test efficiency, reduces time, effort and cost for the test and is very useful in the medicine, especially clinical test.

While the present invention has been described with reference to what are presently considered to be the preferred embodiments, it is to be understood that the invention is not limited to the disclosed embodiments. On the contrary, the invention is intended to cover various modifications and equivalent arrangements included within the spirit and scope of the appended claims. The scope of the following claims is to be accorded the broadest interpretation so as to encompass all such modifications and equivalent structures and functions.

### FREE TEXT OF SEQUENCE LISTING

SEQ ID NO:1 to 60 - Description of Artificial Sequence: Synthetic DNA (primer)

## Claims

1. A method for analyzing a nucleic acid having plural possible mutation sites, comprising the steps of:
hybridizing plural primers with the nucleic acid, wherein the primers are complementary to any of the possible mutation sites at the 3' end;
carrying out extension reaction of the hybridized plural primers;
optically detecting a result of the extension reaction; and
determining whether or not the nucleic acid has at least one mutation site based on the result in the step of optical detection,
wherein the step of optical detection comprises determining the presence or absence of the extension by detecting light emission which is generated from at least one of the plural primers without distinction of species of the primers.

2. The method according to claim 1, wherein the step of optical detection comprises carrying out a luminescence reaction with the use of a pyrophosphate generated in the step of extension thereby detect the luminescence reaction as a result of the extension.

3. The method according to claim 1, wherein the luminescence reaction occurs as a result of a reaction of a luciferase with ATP derived from the pyrophosphate.

4. The method according to claim 1, wherein the extension occurs when the nucleic acid is complementary to at least two bases at the 3' end of any of the plural primers.

5. The method according to claim 1, wherein the step of optical detection comprises hybridizing plural probes with one or more products of the extension, and detecting fluorescence from a hybrid between any of the products and any of the probes, and wherein the plural probes are labeled with a single-species fluorophore.

6. The method according to claim 1, wherein the extension products are labeled with a single-species fluorophore, and wherein the step of optical detection comprises detecting fluorescence with the emission wavelength of the single-species fluorophore.

7. The method according to claim 1, wherein the step of optical detection comprises allowing the extension products to react with an intercalator, and detecting fluorescence derived from the intercalator.

8. The method according to claim 1, wherein the step of hybridization comprises changing proportions of the plural primers in a primer mixture.

9. The method according to claim 8, wherein the proportions of the plural primers are controlled so as to yield substantially the same emission intensity even when any of the plural primers is hybridized with the nucleic acid.

10. An apparatus for analyzing a nucleic acid, comprising:
a reactor for housing at least a sample nucleic acid and plural primers;
a mechanism for controlling the temperature of the reactor;
a detecting unit for detecting light emission in the reactor; and
a control unit for analyzing the result detected by the detecting unit,
wherein the detecting unit is so configured as to detect an extension of any of the plural primers hybridized to the sample nucleic acid as light emission without distinction of species of the primers and to determine the presence or absence of any mutation of the sample nucleic acid based on a result in the detection.

11. A kit for analyzing a nucleic acid, comprising:
plural primers which are complementary to any of plural possible mutation sites of a target nucleic acid at the 3' end, respectively;
one or more enzymes for extending the primers; and
a light-emitting reagent for optically detecting extension of any of the primers,
wherein the plural primers each have a sequence corresponding to any of the mutations.

12. The kit according to claim 11, wherein the plural primers comprise at least one selected from primers listed in Table 1 (SEQ ID NO: 1 to 60).
